# EUROPÄISCHE PATENTSCHRIFT

(11) **EP 0 291 888 B1**
(45) Veröffentlichungstag und Bekanntmachung des Hinweises auf die Patenterteilung: **28.07.1993**
(21) Anmeldenummer: 88107762.2
(22) Anmeldetag: 13.05.1988
(51) Int. Cl.: C07D 487/04, A61K 31/55

(54) **4-substituierte 10-Cyanmethylenpyrrolo[4,3-e]-benzo-azepine**
4-Substituted 1-cyanomethylene pyrrolo[4,3-e]-benzo-azepins
10-Cyanométhylène pyrrolo[4,3-e]-benzo-azépines substituées sur la position 4

(30) Priorität: 21.05.1987 DE 3717068
(43) Veröffentlichungstag der Anmeldung: 23.11.1988
(73) Patentinhaber: BASF Aktiengesellschaft, 67063 Ludwigshafen (DE)
(72) Erfinder: Steiner, Gerd, Dr., D-6719 Kirchheim (DE); Pfister, Juergen, Dr., D-6710 Frankenthal (DE); Teschendorf, Hans-Juergen, Dr., D-6724 Dudenhofen (DE); Unger, Liliane, Dr., D-6700 Ludwigshafen (DE); Binder, Rudolf, Dr., D-6520 Worms (DE)

(56) Entgegenhaltungen:
- EP-A- 0 019 172
- EP-A- 0 050 212
- EP-A- 0 209 022
- E. SCHROEDER et al.: "ARZNEIMITTELCHEMIE I", 1976, Seiten 24-38, Georg Thieme Verlag Stuttgart, D

## Beschreibung

### Beschreibung

Die Erfindung betrifft in 4-Stellung substituierte 10-Cyanmethylen-pyrrolo[4,3-e]-benzo-azepine, Verfahren zu ihrer Herstellung und ihre Anwendung als Arzneimittel, die als Sedativa, Hvpnotika, Tranquilizer, Muskelrelaxantien, Neuroleptika oder Antiparkinsonmittel verwendet werden können.

Es ist bekannt, daß tricyclische Ringsysteme mit einer Dibenzo-Struktur zu einem zentralen heterocyclischen 7-Ring, der gegebenenfalls einen N-Methylpiperazinrest aufweist, neuroleptische Wirkungen aufweisen können. Solche Tricyclen sind beispielsweise N-Methylpiperazin-Derivate von Dibenzo[b,e,][1,4]-diazepinen (Clozapine), Dibenzo[b,f][1,4]-thiazepinen (Clotiapine), Dibenzo-[b,f][1,4]-oxazepinen (Loxapine) oder Morphantridinen (Perlapine), wie beispielsweise aus der Zusammenfassung von J. Schmutz in der Arzneimittelforschung 25, 712-720 (1975) hervorgeht.

In der EP-A-0 019 172, der EP-A-0 050 212 und der EP-A-0 209 022 werden 6-substituierte 11-Alkylen-morphantridine, 5-substituierte-9-Cyanmethylen-dithieno[3,4-b:4',3'-e]azepine und 4-substituierte 10-Cyanmethylen-thieno[4,3-e]-benzo-azepine mit wertvollen pharmakologischen Eigenschaften beschrieben.

Es wurde nun gefunden, daß 4-substituierte 10-Cyanmethylen-pyrrolo[4,3-e]-benzo-azepine der Formel I 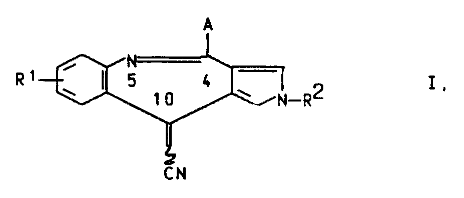
in der
- R¹: ein Wasserstoff- oder Halogenatom,
- R²: einen C₁-C₃-Alkylrest und
- A: einen Piperazinrest, der durch eine C₁-C₃-Alkylgruppe substituiert sein und in Form des N-Oxids vorliegen kann,
bedeuten,
und ihre physiologisch verträglichen Säureadditionssalze wertvolle pharmakologische Eigenschaften aufweisen.

Für den Rest R¹ kommen insbesondere folgende Bedeutungen in Betracht: Wasserstoff, Fluor und Chlor.
- A: ist vorzugsweise ein Piperazinrest.

Besonders bevorzugte Reste für A sind der 4-Methyl-piperazin- und 4-Methyl-4-oxy-piperazinylrest.

Es sei darauf hingewiesen, daß die erfindungsgemäßen Verbindungen der Formel I als (E)- bzw. (Z)-Isomere Ia und b vorliegen.

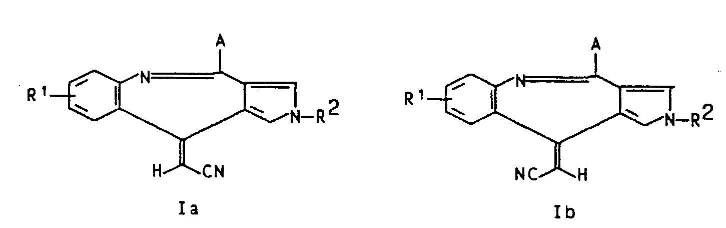

Die (E),(Z)-Isomeren können beispielsweise durch fraktionierte Kristallisation oder durch Säulenchromatographie getrennt werden.

Die Zuordnung der einzelnen Isomeren erfolgt beispielsweise durch Röntgenstrukturanalyse, wie aus den Beispielen hervorgeht.

Die folgenden Verbindungen sind als besonders bevorzugt zu nennen:
(E),(Z)-10-Cyanmethylen-4-(4-methyl-piperazin-1-yl)-pyrrolo[4,3-e]benzo-azepin
(Z)-10-Cyanmethylen-4-(4-methyl-piperazin-1-yl)-pyrrolo[4,3-e]benzo-azepin
(E)-10-Cyanmethylen-4-(4-methyl-piperazin-1-yl)-pyrrolo[4,3-e]benzo-azepin
(E),(Z)-7-Chlor-10-cyanmethylen-(4-methyl-piperazin-1-yl)-pyrrolo[4,3-e]-benzo-azepin.

Die erfindungsgemäßen Verbindungen der Formel I werden hergestellt, indem man eine Verbindung der Formel II 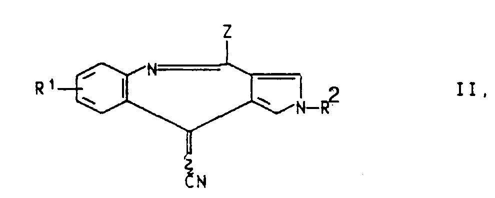
in der R¹ und R² die angegebenen Bedeutungen haben und Z eine nukleofuge Abgangsgruppe darstellt, mit einem Nukleophil AH, in dem A die für Formel I angegebenen Bedeutungen hat, umsetzt, gegebenenfalls in das reine (E)- und (Z)-Isomere trennt und/oder gegebenenfalls die erhaltene Verbindung in das N-Oxid und/oder in das Säureadditionssalz einer physiologisch verträglichen Säure überführt.

Als nukleofuge Abgangsgruppe für Z kommen vorzugsweise Halogenatome, insbesondere Brom oder Chlor, in Betracht.

Die Umsetzung erfolgt zweckmäßig in Gegenwart einer überschüssigen Menge des verwendeten Amins AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Ether, insbesondere Tetrahydrofuran oder Dioxan, von Benzol oder eines Benzolkohlenwasserstoffes, wie Toluol, xylol, Mesitylen oder Decahydronaphthalin oder eines aprotischen polaren Lösungsmittels, wie Dimethylformamid, gearbeitet werden. Falls nur mit einem Äquivalent des Amins AH gearbeitet wird, muß noch 1 Äquivalent einer inerten Base, wie z.B. Triethylamin, zugesetzt werden.

Die Umsetzung erfolgt in der Regel bei Temperaturen von 80 bis 150°C, und ist im allgemeinen innerhalb von 1 bis 10 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2- bis 20-fachen molarem Überschuß verwendet.

Die Überführung einer Verbindung der Formel I in das N-Oxid erfolgt in üblicher Weise, zweckmäßig mit wäßrigem Wasserstoffperoxid (30 Gew.%) in ethanolischer Lösung oder mit einer Peroxysäure in einem Halogenkohlenwasserstoff. Die Überführung in das Säureadditionssalz einer physiologisch verträglichen Säure erfolgt ebenso in üblicher Weise.

Die Ausgangsverbindungen der Formel II werden erhalten, indem man ein 10-Cyanmethylen-4,5-dihydro-pyrrolo[4,3-e]benzo-azepin-4-on der Formel III 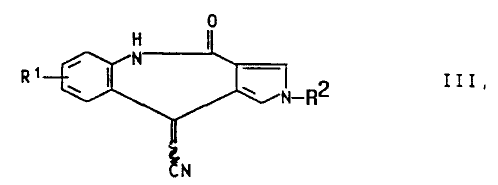
in der R¹ und R² die für die Formel II angegebenen Bedeutungen haben, mit einem Überschuß an Halogenierungsmittel, wie Phosphoroxychlorid, in Gegenwart eines Lösungsmittels, bevorzugt in einem Halogenkohlenwasserstoff und in Gegenwart einer katalytischen Menge N,N-Dimethylanilin 1 bis 5 Stunden auf Rückflußtemperatur erhitzt und das erhaltene Iminochlorid nach dem Abdestillieren des überschüssigen Phosphoroxychlorids und Aufarbeitung in einem wäßrigen zweiphasigen System durch Extraktion mit einem chlorierten Kohlenwasserstoff, wie Methylenchlorid, isoliert.

Das neue 10-Cyanmethylen-4,5-dihydro-pyrrolo[4,3-e]benzo-azepin-4-on der Formel III, in der R¹ und R² die für die Formel I angegebenen Bedeutungen haben, wird durch Carbonyl-Olefinierung hergestellt, indem man ein 4,5-Dihydro-pyrrolo[4,3-e]benzo-azepin-4,10-dion der Formel IV 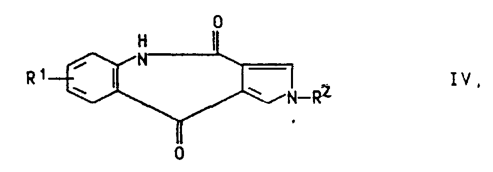
mit einem Phosphonat der Formel Va 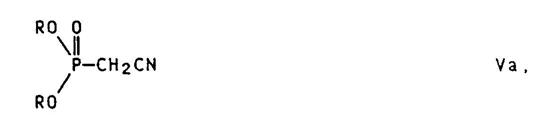
in der R einen Alkylrest mit 1 bis 3 C-Atomen bedeutet, unter den Bedingungen der Wittig-Horner-Reaktion in Dimethylformamid und in Gegenwart von einem Moläquivalent Natrium-t-butylat, bei Temperaturen von 20 bis 100°C umsetzt oder mit einem Phosphoniumsalz der Formel Vb 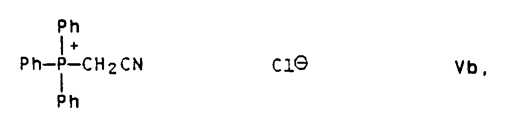
in der Ph für einen Phenylrest steht, unter den Bedingungen der klassischen Wittig-Reaktion in Dimethylformamid in Gegenwart von einem Moläquivalent Natriumethylat bei Temperaturen von 20 bis 100°C umsetzt.

Das neue 4,5-Dihydro-pyrrolo[4,3-e]benzo-azepin-4,10-dion der Formel IV, in der R¹ und R² die für die Formel I angegebenen Bedeutungen haben, wird durch Friedel-Crafts-Cyclisierung hergestellt, indem man ein Pyrrol-3,4-dicarbonsäure-benzamid der Formel VI 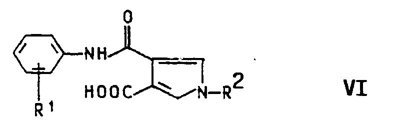
in Gegenwart einer starken Säure, vorzugsweise in Polyphosphorsäure, die zugleich als Solvens dient, bei Temperaturen zwischen 20 und 150°C wahrend 1 bis 3 Stunden cyclisiert.

Das Pyrrol-3,4-dicarbonsäure-benzamid der Formel VI erhält man in einfacher Weise durch Umsetzung des Pyrrol-3,4-dicarbonsäureanhydrids mit dem entsprechenden Anilin in einem inerten organischen Lösungsmittel, wie z.B. Methylenchlorid, bei Raumtemperatur während 1 bis 5 Stunden.

Die Pyrrol-3,4-dicarbonsäureanhydride entstehen aus den literaturbekannten Pyrrol-3,4-dicarbonsäuren, die am Stickstoff den Substituenten R² besitzen (Bull. Soc. Chim. Fr. 1975, 2335; J. Heterocycl. Chem. 1983, 20(2), 321) durch Erhitzen mit Acetanhydrid auf Temperaturen zwischen 20 und 150°C in an sich bekannter Weise.

Die erfindungsgemäßen Verbindungen der Formel I werden in der Regel in Form gelblicher bis gelber Kristalle erhalten und können durch Umkristallisation aus den üblichen organischen Lösungsmitteln, bevorzugt aus einem niederen Alkohol, wie Ethanol, umkristallisiert oder durch Säulenchromatographie gereinigt werden.

Falls erforderlich erfolgt eine Trennung in die einzelnen (E)- und (Z)-Isomeren durch fraktionierte Kristallisation in einem chlorierten Kohlenwasserstoff, bevorzugt Methylenchlorid, einem niederen einwertigen Alkohol, bevorzugt Methanol oder Ethanol, oder einem gesättigten cycloaliphatischen Kohlenwasserstoff, bevorzugt Cyclohexan, oder durch Säulenchromatographie an Kieselgel insbesondere mit Methylenchlorid und Methanol im Verhältnis 99:1 bis 85:15 Volumenteilen.

Die freien substituierten 10-Cyanmethylen-pyrrolo[4,3-e]benzo-azepine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure und Zitronensäure.

Die erfindungsgemäßen Verbindungen weisen wertvolle pharmakologische Eigenschaften auf. Sie können als Sedativa, Hypnotika, Tranquilizer, Muskelrelaxantien, Neuroleptika oder Antiparkinsonmittel Verwendung finden. Mehrere der genannten Wirkungsqualitäten können kombiniert bei einer erfindungsgemäßen Verbindung auftreten. In manchen Fällen kann das einzelne reine Isomere nach erfolgter Isomerentrennung eine Wirkung bevorzugt aufweisen. Die neuen Substanzen eignen sich daher zur Behandlung psychischer Störungen, insbesondere Schizophrenie, Angst, Unruhezustände sowie Störungen des extrapyramidalmotorischen Systems, z.B. Morbus Parkinson.

Zur Wirkungscharakterisierung wurden folgende Methoden angewendet:

### 1. Sedative Wirkung

4 bis 8 Gruppen von jeweils 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min.

Als ED₅₀ wird die Dosis bestimmt, welche im Vergleich zu placebobehandelten Kontrolltieren eine Abnahme der Orientierungshypermotilität um 50 % bewirkt.

### 2. L-5-HTP-Antagonismus (Serotonin-Antagonismus)

L-5-hydroxytryptophan (L-5-HTP), ein pro-drug für Serotonin, führt an der Ratte (316 mg/kg i.p.) zu einem Erregungsbild, das durch Symptome wie Kopfschütteln, Tremor und Vorderpfotenbewegungen charakterisiert ist. Die Tiere werden 1 h lang nach L-5-HTP-Gabe beobachtet und die auftretenden Symptome alle 10 min mit Hilfe eines Score quantifiziert. Die Testsubstanzen werden 1 h vor L-5-HTP p.o. gegeben, n/Dosis = 6. Als ED₅₀ wird die Dosis berechnet, die im Mittel die bei einem Kontrollkollektiv beobachteten Scorewerte um 50 % reduziert.

### 3. Anticholinerge Wirkung

Gruppen von 10 weiblichen NMRI-Mäusen erhalten Physostigmin in einer letalen Dosis (0,825 mg/kg) subcutan. Die Prüfsubstanzen werden 30 min vor der Physostigminapplikation oral appliziert.
Als ED₅₀ wird die Substanzdosis bestimmt, welche 50 % der Tiere vor dem Tod durch Physostigmin schützt.

### 4. In vitro-Bestimmung der Affinität zu den Dopamin-D₁- und D₂-Rezeptoren mit Hilfe von Kompetitionsversuchen

In Anlehnung an die Methode von Seeman et al. (J. Neurochem. 43, 221-235, 1984) wurden 1 ml Ansätze mit Nucleus-caudatus-Membranen vom Rind in 50 mM Tris-HCl-Puffer pH 7,4 mit 120 mM NaCl, 5 mM KCl, 1 mM MgCl₂ x 6 H₂O und 2 mM CaCl₂ x 2 H₂O sowie mit steigenden Konzentrationen von Prüfsubstanz und einer festen Konzentration des Dopamin-D₁- oder -D₂-Rezeptorliganden ³H-SCH 23390 (1 nM) bzw. ³H-Spiperon (0,2 nM) hergestellt. Die unspezifische Bindung wurde mit 10 µM (+)-Butaclamol bzw. mit 1 µM Haloperidol bestimmt. Nach 60 min Inkubation bei 30°C wurden die Ansätze über Glasfaserfilter (GF/B, Whatman) filtriert und die Menge des auf dem Filter zurückgehaltenen Radioliganden mittels Flüssigkeitsszintillationsmessung bestimmt.

Die Kompetitionskonstanten (Kᵢ-Werte) wurden durch nichtlineare Regressionsanalyse an einem IBM-Rechner in Anlehnung an das Programm "Ligand" von Munson und Rodbard (Anal. Biochem. 107, 220, 1980) berechnet.

**Tabelle 1**

| Substanz des Beispiels | Sedative Wirkung Maus ED 50 mg/kg p.o. | Antiserotoninerge Wirkung Ratte ED 50 mg/kg p.o. | Anticholinerge Wirkung Maus ED 50 mg/kg p.o. |
|---|---|---|---|
| 1a | 2,96 | 1,18 | > 21,5 |
| 3 | 0,82 | 0,2 | 17,5 |
| 3a | 0,67 | 0,29 | |
| 5 | 4,7 | 3,3 | 16,7 |
| Clozapine | 3,8 | 6,3 | 14,1 |

**Tabelle 2**

| Substanz des Beispiel | Affinität zum | |
|---|---|---|
| | D 1-Rezeptor Hemmung der ³H-SCH 23 390-Bindung Ki (nM) | D 2-Rezeptor Hemmung der ³H-Spiperon-Bindung Ki (nM) |
| 1a | 10,5 | ca. 2000 |
| 3 | 20,0 | ca. 1000 |
| 5 | 20,8 | ca. 2000 |
| Clozapine | 172 | 270 |

In den durchgeführten Untersuchungen, in denen Sedativa, minor und major Tranquillizer typischerweise Wirkungen zeigen, finden sich für die erfindungsgemäßen Verbindungen gute Wirkungen, wobei die Wirkung der Referenzsubstanz Clozapine meist erreicht oder übertroffen wird. Dies gilt für Beispiel 1a und 3 bei der sedativen Wirkung und für die Beispiele 1a. 3, 5 bei der antiserotoninergen Wirkung.

Ein Unterschied im Vergleich zu Clozapin, und damit ein neues Wirkungsprofil. ergibt sich bei der antidopaminergen Wirkung, die biochemisch als Affinität zum D1-Rezeptor und zum D2-Rezeptor gemessen wurde. Während bei Clozapine die Affinität zum D1-Rezeptor gering über der zum D2-Rezeptor liegt, sind die erfindungsgemäßen Substanzen selektive D1-Antagonisten. Die Ki-Werte für die Verdrängung vom D1-Rezeptor liegen ca. 50 mal tiefer als die Ki-Werte für die Verdrängung vom D2-Rezeptor. Weiterhin wird die D1-antagonistische Wirkstärke von Clozapine durch die neuen Substanzen deutlich übertroffen (Faktor 9 bis 16).

Die anticholinerge Wirkung ist weniger ausgeprägt, was im Hinblick auf dadurch bedingte klinische Nebenwirkungen vorteilhaft ist.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 20 mg/kg Körpergewicht bei oraler Gabe und zwischen 0,1 und 2 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die folgenden Beispiele dienen zur Erläuterung der Erfindung:

### Beispiel 1

### A Herstellung der Ausgangsstoffe

### a) N-Methyl-pyrrol-3,4-dicarbonsäureanhydrid

30,4 g (180 mM) N-Methyl-pyrrol-3,4-dicarbonsäure wurden mit 250 ml Essigsäureanhydrid 1 h unter Rückfluß gekocht. Über einen Kolonnenkopf wurden anschließend während 4 h insgesamt ca. 200 ml Essigsäureanhydrid im Gemisch mit Essigsäure abdestilliert. Nach Filtrieren engte man den Ansatz bis zur Trockene ein, versetzte mit 50 ml Toluol und engte wiederum zur Trockene ein. Nach nochmaliger Wiederholung dieser Operation isolierte man 37,6 g Produkt als Kristall-Öl-Gemisch, das noch etwas Acetanhydrid enthält, aber für die weitere Umsetzung genügend rein ist.

### b) N-Methyl-pyrrol-3,4-dicarbonsäure-monobenzamid

Zu 20,0 g (132 mM) N-Methyl-pyrrol-3,4-dicarbonsäureanhydrid in 80 ml Methylenchlorid wurden 16,0 g (172 mM) Anilin in 20 ml Methylenchlorid bei Raumtemperatur unter gutem Rühren zugetroft. Man ließ anschließend 5 h nachrühren, saugte dann die dick ausgefallenen Festkörper ab, wusch gut mit Methylenchlorid nach und trocknete die Festkörper zuerst an der Luft und später im Vakuum. Die Ausbeute betrug 19,8 g (61 %). Schmp. 248-250°C.

### c) 2-Methyl-4,5-dihydro-pyrrolo[4,3-e]benzo-azepin-4,10-dion

19,2 g (79 mM) N-Methyl-pyrrol-3,4-dicarbonsäure-mono-benzamid wurde portionsweise in 240 g Polyphosphorsäure unter gutem Rühren bei 100°C Innentemperatur eingetragen und 1 h bei 115-120°C Innentemperatur nachgerührt. Nach dem Abkühlen goß man den Ansatz auf Eis/Wasser und ließ 1 h nachrühren. Nach dem Stehen über Nacht saugte man das feine hellbraune Rohprodukt ab, wusch gut mit Wasser nach und trocknete im Vakuumtrockenschrank bei 60°C. Man isolierte 13,4 g (75 %) Produkt, Schmp. > 280°C, das für die weitere Umsetzung genügend rein ist.

### d) (E),(Z)-10-Cyanmethylen-2-methyl-4,5-dihydro-pyrrolo[4,3-e]benzo-azepin-4-on ((E),(Z)-Isomerengemisch)

Zur Herstellung dieses Produktes führte man eine Carbonyl-Olefinierung des 2-Methyl-4,5-dihydro-pyrrolo[4,3-e]benzo-azepin-4,10-dions mit Hilfe der Wittig-Horner-Reaktion (α) oder über die klassische Wittig-Synthese (β) durch:
(α) 9,6 g (43 mM) 2-Methyl-4,5-dihydro-pyrrolo[4,3-e]benzo-azepin-4,10-dion wurden in 70 ml Dimethylformamid unter Erwärmen auf 60-70°C gelöst und unter Stickstoff gerührt. Man ließ dann 15,0 g (85 mM) Diethyl-cyanmethyl-phosphonat zutropfen und setzte portionsweise 9,5 g (85 mM) Kalium-tertiärbutylat bei 60-70°C Innentemperatur unter gutem Rühren zu. Anschließend ließ man noch 2 h bei 80°C nachrühren.
   Nach Entfernen des Lösungsmittels im Vakuum nahm man den Rückstand in 400 ml Eis/Wasser auf, säuerte mit wenig 10-proz. Salzsäure an, ließ 1 h unter Kühlung nachrühren und saugte das hellbraune Rohprodukt unter reichlichem Nachwaschen mit Wasser ab. Nach Trocknen im Vakuum bei 60°C erhielt man 10,2 g (95 %) Produkt, Schmp. 277-280°C.
(β) Man legte Triphenyl-cyanomethyl-phosphonium-chlorid in Dimethylformamid vor, ließ anschließend 1 Moläquivalent einer 30%igen Natriummethylat-Lösung zutropfen oder versetzte mit 1 Moläquivalent Natriumhydrid und fügte zuletzt 1 Moläquivalent einer Lösung von 2-Methyl-4,5-dihydro-pyrrolo[4,3-e]benzo-azepin-4,10-dion in Dimethylformamid hinzu. Man ließ das Reaktionsgemisch 5 bis 8 h bei 50 bis 80°C nachrühren, goß anschließend auf Eiswasser und extrahierte mehrmals mit Methylenchlorid. Nach dem Trocknen und Einengen der organischen Phase erhielt man das Rohprodukt. Ausbeute: 67 % farblose Kristalle, Schmp. 275-279°C.

### B Herstellung des Endprodukts

### (E)- und (Z)-10-Cyanmethylen-2-methyl-4-(4-methyl-piperazin-1-yl)-pyrrolo[4,3-e]-benzo-azepin

a) 5,8 g (23 mM) 10-Cyanmethylen-2-methyl-4,5-dihydro-pyrrolo[4,3-e]-benzo-azepin-4-on ((E),(Z)-Isomerengemisch) in 70 ml 1,1,1-Trichlorethan wurden mit 20 ml Phosphoroxychlorid und 0,2 ml N,N-Dimethylanilin versetzt und 1 h unter Stickstoffatmosphäre unter Rückfluß gekocht. Nach vollständigem Abdestillieren des überschüssigen Phosphoroxychlorids und Dimethylanilins im Ölpumpenvakuum verteilte man den Rückstand zwischen Methylenchlorid und Wasser, filtrierte das Gemisch, extrahierte die wäßrige Phase noch zweimal mit Methylenchlorid und wusch die vereinigten organischen Phasen mit verdünnter Salzsäure und Wasser gründlich nach. Trocknen und Einengen der organischen Phase lieferte 5,8 g (95 %) 4-Chlor-10-cyanmethylen-2-methyl-pyrrolo[4,3-e]benzo-azepin, das für die weitere Umsetzung genügend rein ist.
   5,8 g (22 mM) 4-Chlor-10-cyanmethylen-2-methyl-pyrrolo[4,3-e]benzo-azepin wurden in 50 ml Dimethylformamid gelöst, mit 6,6 ml (60 mM) N-Methylpiperazin (stark exotherme Reaktion) versetzt und 1 h bei 80°C unter Stickstoff gerührt. Nach Entfernen des Lösungsmittels im Vakuum nahm man den Rückstand in ca. 200 ml Eis/Wasser auf, stellte mit wenig 10-proz. Natronlauge alkalisch, ließ 1 h unter Kühlung nachrühren und saugte das hellbraune Rohprodukt unter reichlichem Nachwaschen mit Wasser ab. Das Rohprodukt reinigte man durch Säulenchromatographie (Kieselgel, Laufmittel Methylenchlorid/Methanol 99/5). Man erhielt 5,3 g (73 %) gelbliches 10-Cyanmethylen-2-methyl-4-(4-methylpiperazin-1-yl)-pyrrolo[4,3-e]benzo-azepin in Form eines (E),(Z)-Isomerengemisches, das aus Isopropanol umkristallisiert wurde: Schmp. 178-180°C.
b) Zur Trennung der (E),(Z)-Isomeren reicherte man das Isomerengemisch durch Säulen-chromatographie (Kieselgel, Laufmittel Methylenchlorid/Methanol 95/5) in der unpolaren und polaren Fraktion an und kristallisierte jeweils die beiden angereicherten geometrischen Isomeren aus Isopropanol fraktioniert um. Dabei fielen die (E),(Z)-Isomeren praktisch rein an.
   Das (E)-Isomere a wurde als unpolare Komponente (Dünnschichtchromatographie, Kieselgel, Laufmittel Toluol/Methanol 85/15) in einer Ausbeute von 1,8 g mit dem Schmp. 208-210°C isoliert.

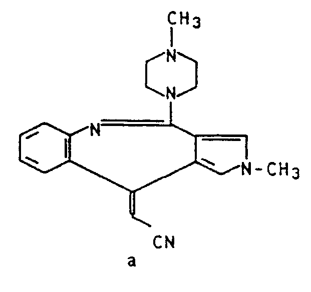

### Beispiel 2

### (E),(Z)-10-Cyanmethylen-2-methyl-4-(4-methyl-4-oxy-piperazin-1-yl)-pyrrolo-[4,3-e]benzo-azepin · 2,5 H₂O

2,0 g (6,0 mM) cis, trans-10-Cyanmethylen-2-methyl-4-(4-methyl-piperazin-1-yl)-pyrrolo-[4,3-e]benzo-azepin (vgl. Beispiel 1) wurden in 100 ml Methylenchlorid gelöst und mit 1,3 g (6,0 nM) 3-Chlorperoxybenzoesäure versetzt. Nach einstündigem Nachrühren bei Raumtemperatur verdünnte man den Ansatz mit 100 ml Methylenchlorid, fügte ca. 100 ml H₂O hinzu und stellte mit 10 % Natronlauge auf pH = 12. Die wäßrige Phase extrahierte man noch einige Male mit Methylenchlorid, trocknete die vereinigten organischen Phasen, engte ein und reinigte das erhaltene N-Oxid durch Säulenchromatographie (Kieselgel, Laufmittel Methylenchlorid/Methanol 1/1). Man isolierte 1,2 g (58 %) gelbe Kristalle, Schmp. 158-160°C.

Die folgenden Substanzen erhält man analog Beispiel 1 und 2 unter Einsatz der entsprechenden substituierten Ausgangsverbindungen:
3. (E).(Z)-7-Chlor-10-cyanmethylen-2-methyl-4-(4-methyl-piperazin-1-yl)-pyrrolo-[4,3-e]benzo-azepin,
3a. (E)-7-Chlor-10-cyanmethylen-2-methyl-4-(4-methyl-piperazin-1-yl)-pyrrolo-[4,3-e]benzo-azepin,
4. (E),(Z)-7-Fluor-10-cyanmethylen-2-methyl-4-(4-methyl-piperazin-1-yl)-pyrrolo-[4,3-e]benzo-azepin,
5. (E)-(Z)-10-Cyanmethylen-2-methyl-4-(4-ethyl-piperazin-1-yl)-pyrrolo[4,3-e]benzo-azepin.

### Beispiel 6

Auf einer Tablettenpresse werden in üblicher Weise Tabletten folgender Zusammensetzung gepreßt:
40 mg Substanz des Beispiels 1 (E)
120 mg Maisstärke
13,5 mg Gelatine
45 mg Milchzucker
2,25 mg Aerosil^{®} (chemisch reine Kieselsäure in submikroskopisch feiner Verteilung)
6,75 mg Kartoffelstärke (als 6%iger Kleister)

### Beispiel 7

In üblicher Weise werden Dragees folgender Zusammensetzung hergestellt:
20 mg Substanz des Beispiels 1 (E)
60 mg Kernmasse
60 mg Verzuckerungsmasse
Die Kernmasse besteht aus 9 Teilen Maisstärke, 3 Teilen Milchzucker und 1 Teil Luviskol^{®} VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Beispiel 8

10 g Substanz des Beispiels 1 (E) werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche (Patentansprüche für folgende(n) Vertragsstaat(en): AT, BE, CH, DE, FR, GB, IT, LI, NL)

1. 4-Substituierte 10-Cyanmethylen-pyrrolo[4,3-e]benzo-azepine der Formel I 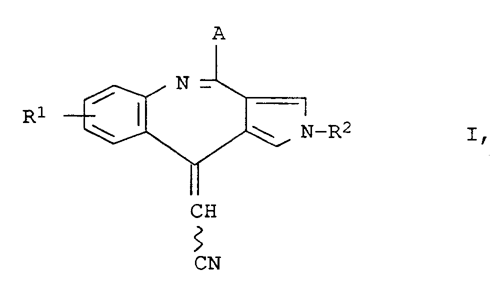 worin
R¹ ein Wasserstoff- oder Halogenatom,
R² eine C₁₋₃-Alkylgruppe und
A einen Piperazinrest, der durch eine C₁₋₃-Alkylgruppe substituiert sein kann und in Form des N-Oxids vorliegen kann,
bedeuten, sowie deren Salze mit physiologisch verträgliche Säuren.

2. Verbindungen der Formel I gemäß Anspruch 1, dadurch gekennzeichnet, daß
R¹ ein Wasserstoff-, Fluor- oder Chloratom,
R² eine Methylgruppe und
A einen N-Methylpiperazinrest bedeutet.

3. Verfahren zur Herstellung von Verbindungen der Formel I gemäß Anspruch 1, dadurch gekennzeichnet, daß man eine Verbindung der Formel II 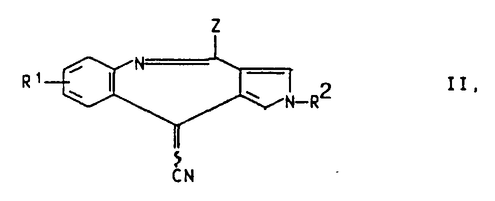 in der R₁ und R₂ die für Formel I angegebenen Bedeutungen haben und Z eine nukleofuge Abgangsgruppe darstellt, mit einem Amin AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

4. Verbindungen der Formel I gemäß Anspruch 1 zur Verwendung bei der Bekämpfung von Krankheiten.

## Patentansprüche (Patentansprüche für folgende(n) Vertragsstaat(en): ES)

1. Verfahren zur Herstellung von 4-substituierten 10-Cyanmethylen-pyrrolo[4,3-e]benzo-azepinen der Formel I 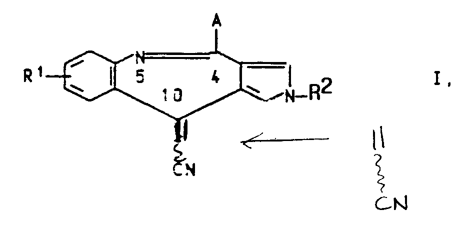 in der
R¹ ein Wasserstoff- oder Halogenatom,
R² eine C₁-C₃-Alkylgruppe und
A einen Piperazinrest, der durch eine C₁-C₃-Alkylgruppe substituiert sein und in Form seines N-Oxids vorliegen kann,
bedeuten, und deren physiologisch verträglichen Säureadditionssalzen, dadurch gekennzeichnet, daß man eine Verbindung der Formel II 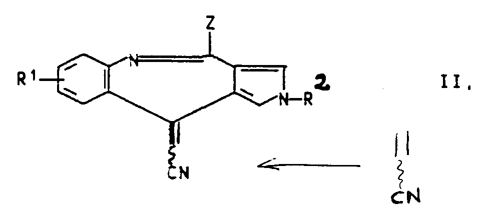 in der R¹ und R² die für Formel I angegebenen Bedeutungen haben und Z eine nukleofuge Abgangsgruppe darstellt, mit einem Amin AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

2. Verfahren gemäß Anspruch 1, dadurch gekennzeichnet, daß man Verbindungen der Formel I herstellt, in denen R¹ Wasserstoff, Chlor oder Fluor und R² eine Methylgruppe bedeuten und A für einen N-Methylpiperazinrest steht.

## Claims (Claims for the following Contracting State(s): AT, BE, CH, DE, FR, GB, IT, LI, NL)

1. A 4-substituted 10-cyanomethylenepyrrolo[4,3-e]-benzazepine of the formula I 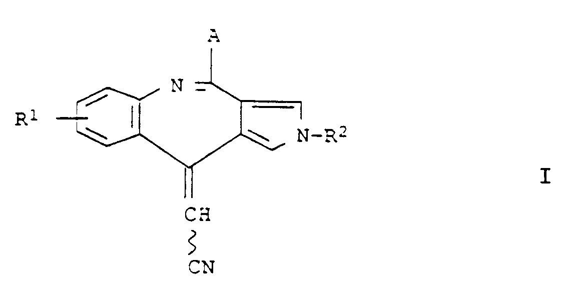 where
R¹ is hydrogen or halogen,
R² is C₁₋₃-alkyl and
A is a piperazine residue which can be substituted by C₁₋₃-alkyl and can be in the form of an N-oxide,
and the salts thereof with physiologically tolerated acids.

2. A compound of the formula I as claimed in claim 1, wherein
R¹ is hydrogen, fluorine or chlorine,
R² is methyl and
A is an N-methylpiperazine residue.

3. A process for the preparation of a compound of the formula I as claimed in claim 1, which comprises reacting a compound of the formula II 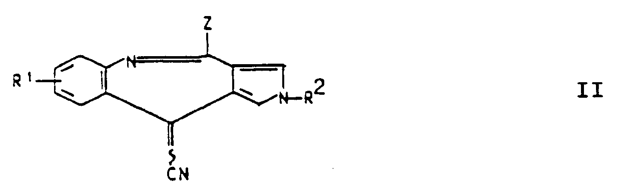 where R¹ and R² have the meanings stated for formula I, and Z is a nucleofugic leaving group, with an amine AH in which A has the meanings stated for formula I, and, where appropriate, converting the resulting compound to the N-oxide and/or into the acid addition salt of a physiologically tolerated acid.

4. A compound of the formula I as claimed in claim 1 for use for controlling diseases.

## Claims (Claims for the following Contracting State(s): ES)

1. A process for the preparation of a 4-substituted 10-cyanomethylenepyrrolo[4,3-e]benzazepine of the formula I 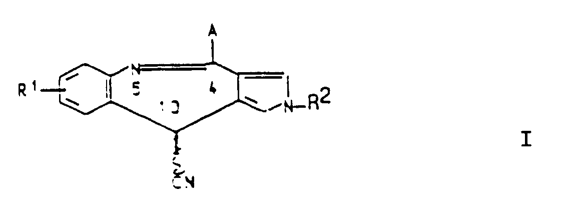 where R¹ is hydrogen or halogen,
R² is C₁-C₃-alkyl and
A is a piperazine residue which can be substituted by C₁-C₃-alkyl and can be present in the form of N-oxide thereof,
and the physiologically tolerated acid addition salts thereof, which comprises reacting a compound of the formula II 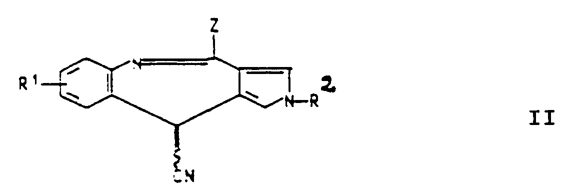 where R¹ and R² have the meanings stated for formula I, and Z is a nucleofugic leaving group, with an amine AH in which A has the meanings stated for formula I, and, where appropriate, converting the resulting compound to the N-oxide and/or into the acid addition salt of a physiologically tolerated acid.

2. A process as claimed in claim 1, wherein a compound of the formula I where R¹ is hydrogen, chlorine or fluorine and R² is methyl, and A is an N-methylpiperazine residue is prepared.

## Revendications (Revendications pour l'(les) Etat(s) contractant(s) suivant(s): AT, BE, CH, DE, FR, GB, IT, LI, NL)

1. 10-Cyanométhylène-pyrrolo[4,3-e]benzo-azépines substituées en position 4, de la formule I 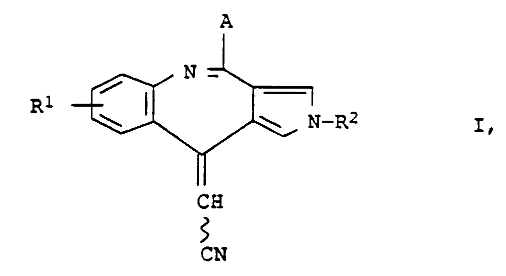 dans laquelle
R¹ représente un atome d'hydrogène ou un atome d'halogène,
R² représente un radical alkyle en C₁-C₃ et
A représente un reste de pipérazine. qui peut être substitué par un radical alkyle en C₁-C₃ et peut se présenter sous la forme du N-oxyde,
comme aussi leurs sels avec des acides physiologiquement compatibles.

2. Composés de la formule I selon la revendication 1, caractérisés en ce que
R¹ représente un atome d'hydrogène, un atome de fluor ou un atome de chlore,
R² représente le radical méthyle et
A représente un reste de N-méthylpipérazine.

3. Procédé de préparation de composés de la formule I selon la revendication 1, caractérisé en ce que l'on fait réagir un composé de la formule II 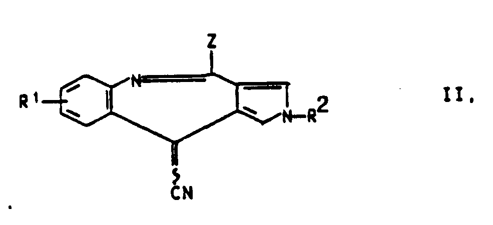 dans laquelle R¹ et R² ont les significations qui leur ont été attribuées à propos de la définition de la formule I et Z représente un groupe sortant nucléofuge, avec une amine AH dans laquelle A possède les significations qui lui ont été attribuées à propos de la définition de la formule I et on convertit éventuellement le composé obtenu en le N-oxyde et/ou en le sel d'addition d'acide d'un acide physiologiquement compatible.

4. Composés de la formule I selon la revendication 1, destinés à être utilisés pour la lutte contre des maladies.

## Revendications (Revendications pour l'(les) Etat(s) contractant(s) suivant(s): ES)

1. Procédé de préparation de 10-cyanométhylène-pyrrolo[4,3-e]benzo-azépines substituées en position 4, de la formule I 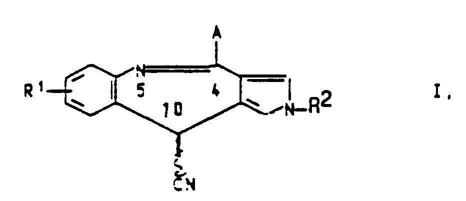 dans laquelle
R¹ représente un atome d'hydrogène ou un atome d'halogène,
R² représente un radical alkyle en C₁-C₃ et
A représente un reste de pipérazine qui peut être substitué par un radical alkyle en C₁-C₃ et peut se présenter sous la forme de son N-oxyde,
et de leurs sels d'addition d'acides physiologiquement compatibles, caractérisé en ce que l'on fait réagir un composé de la formule II 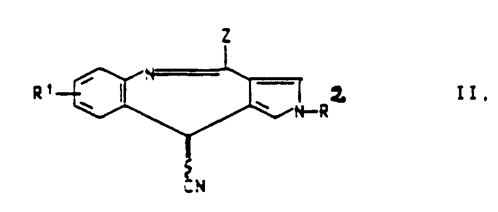 dans laquelle R¹ et R² ont les significations qui leur ont été attribuées à propos de la définition de la formule I et Z représente un groupe sortant nucléofuge, avec une amine AH dans laquelle A possède les significations qui lui ont été attribuées à propos de la définition de la formule I et on convertit éventuellement le composé obtenu en le N-oxyde et/ou en le sel d'addition d'acide d'un acide physiologiquement compatible.

2. Procédé suivant la revendication 1, caractérisé en ce que l'on prépare des composés de la formule I dans laquelle R¹ représente un atome d'hydrogène, un atome de chlore ou un atome de fluor et R² représente le radical méthyle et A représente un reste de N-méthylpipérazine.
